(19) Europäisches Patentamt
European Patent Office
Office européen des brevets

(11) Publication number: **0 396 526 A2**

(12) # EUROPEAN PATENT APPLICATION

(21) Application number: **90870063.6**

(51) Int. Cl.5: **C07C 227/32**

(22) Date of filing: **30.04.90**

(30) Priority: **01.05.89 US 345808**

(43) Date of publication of application:
**07.11.90 Bulletin 90/45**

(84) Designated Contracting States:
**AT BE CH DE DK ES FR GB GR IT LI LU NL SE**

(71) Applicant: **MONSANTO COMPANY**
**Patent Department 800 North Lindbergh Boulevard**
**St. Louis, Missouri 63167(US)**

(72) Inventor: **Talley, John Jeffrey**
**1510 Amisk Court**
**Chesterfield, Mo 63017(US)**

(74) Representative: **Lunt, John Cooper et al**
**Monsanto Europe S.A. Patent Department**
**Avenue de Tervuren 270-272 Letter Box No 1**
**B-1150 Brussels(BE)**

(54) **Method of preparing chiral beta-amino acids.**

(57) The present invention is directed to asymmetric synthesis of β-amino acids wherein Curtius rearrangement of 2(R)-mono-substituted succinates is effected and the incipient isocyanate is trapped with a primary or secondary alcohol. The resulting carbamate-protected β-amino esters are then saponified to produce the corresponding carbamate-protected β-amino acids which are deprotected to yield β-amino acids which possess the same absolute configuration as naturally-occurring (L)-amino acids.

EP 0 396 526 A2

# METHOD OF PREPARING CHIRAL β-AMINO ACIDS

## BACKGROUND OF THE INVENTION

### 1. Field of the Invention

The present invention relates to asymmetric synthesis of β-amino acids and, more particularly, relates to a method of preparing chiral β-amino acids wherein Curtius rearrangement of 2(R)-mono-substituted succinates and trapping of the incipient isocyanate derivative with a primary or secondary alcohol produces chiral carbamate-protected β-amino esters in high yield which are subsequently saponified and deprotected to yield the desired β-amino acid.

### 2. Related Art

β-amino acids are useful replacements in pharmacologically active peptides for the corresponding α-amino acid in order to potentiate the activity of the peptide or to increase resistance thereof to enzymatic degradation. For example, [1-β-alanine]-ACTH (1-18)-octadecapeptide has enhanced and prolonged adrenocorticotropic and lipotropic activities. In addition, substitution of β-amino acids within certain bradykinins have been shown to be resistant to dipeptidylcarboxypeptidase and a number of vasopressin analogues have been made with β-amino acid residues.

Various methods have been proposed for the preparation of chiral β-amino acids. See, for example, Chemistry and Biochemistry of Amino Acids, Vol. 4, Chapter 5, pp. 250-57, B. Weinstein, Ed., Dekker, N.Y. (1975). Furukawa et al, Chem. Pharm. Bull., 25, 1319 (1977), disclose asymmetric synthesis of β-amino acids by addition of chiral amines to carbon-carbon double bonds having nitrile or ester groups in the α-position. However, optical purities of the β-amino acids thus produced range from 2 to 19%. Furukawa et al also report that optically active β-amino acids have been produced with optical purities ranging from 2 to 28% by reacting chiral Schiff bases with Reformsky reagent. Terentev et al, Dohl. Ahad. Nauh SSSR, 163,674 (1965) disclose synthesis of β-aminobutyric acids involving addition of chiral amines to crotonic acid with optical purities ranging from 7-9%.

Brown et al, Tetrahedron Lett., Vol. 28, No. 19, pp 2179-2182 (1987), disclose a method of preparing optically active disubstituted β-amino acids which involves asymmetric catalytic hydrogenation of N-substituted α-(aminoalkyl) acrylates. In order to verify the stereochemistry of the product, Curtius rearrangement was effected on the monomethyl ester of optically enriched RR-anti-2,3-dimethyl-succinic acid and trapping of the incipient isocyanate derivative with a tertiary alcohol, namely, t-butyl alcohol, to give the corresponding R-enriched β-amino acid. Although no yields were reported utilizing t-butyl alcohol, when this Curtius rearrangement was repeated, the yield was only about 23% based on the starting disubstituted succinic acid. Thus, although such method may be useful for verification purposes, from a practical commercial standpoint such method is not acceptable.

Ninomita et al, Tetrahedron Lett., Vol. 30, 2152-2157 (1975) studied the Curtius rearrangement utilizing benzoic acid, diphenylphosphoryl azide and triethylamine followed by treatment with various alcohols and found that t-butyl alcohol gives yields superior to benzyl alcohol, ethanol and phenol.

## BRIEF SUMMARY OF THE INVENTION

It has now been discovered that utilization of a primary or secondary alcohol to trap an isocyanate derivative of a chiral mono-substituted succinate, and, in particular, in a Curtius rearrangement of a chiral mono-substituted succinate, to produce chiral β-amino acids significantly increases the overall yield to an acceptable commercial level.

Accordingly, the present invention is directed to asymmetric synthesis of β-amino acids wherein Curtius rearrangement of 2(R)- mono-substituted succinates is effected and the incipient isocyanate is trapped with a primary or secondary alcohol. The resulting carbamate-protected β-amino esters are then saponified to produce the corresponding carbamate-protected β-amino acids which are then deprotected to produce β-amino acids possessing the same absolute configuration as naturally-occurring (L)-amino acids.

The overall reaction sequence can be shown as follows:

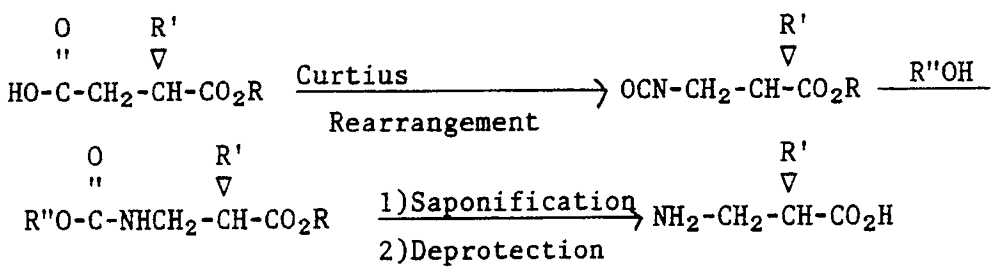


wherein R and R′ independently represent substituted and unsubstituted alkyl radicals having from 1 to about 12 carbon atoms, substituted and unsubstituted cycloalkyl radicals having from about 4 to about 7 carbon atoms, and substituted and unsubstituted aryl, aralkyl and alkaryl radicals, and R″ represents radicals derived from primary and secondary alcohols.

## DETAILED DESCRIPTION OF THE INVENTION

This invention is directed to asymmetric synthesis of β-amino acids represented by the formula:

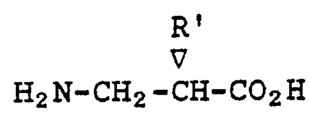

wherein R′ represents radicals as defined above.

The subject method for preparing such compounds involves Curtius rearrangement of 2(R)-mono-substituted succinates represented by the formula

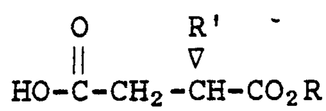

wherein R and R′ are the same as defined above, to afford the isocyanate derivative:

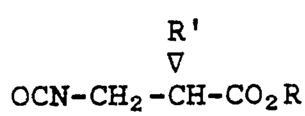

Curtius rearrangement involves pyrolysis of acyl azides $(R-\overset{O}{\overset{\|}{C}}-N=N=N)$ to yield isocyanates $(R-N=C=O)$ which can be subsequently hydrolyzed to give amines. See March, Advanced Organic Chemistry, p. 1005, 2nd ed (1977). As a general rule, Curtius rearrangement is a concerted reaction and therefore proceeds with retention of configuration of the starting materials. Determination of specific reaction conditions for effecting Curtius rearrangements of various succinates is within the skill of one in the art familiar with such reactions. In the method of the present invention, Curtius rearrangement to afford the desired isocyanate is preferably effected by treating a 2(R)-mono-substituted succinate with one equivalent of diphenoxyphosphoryl azide $(PhO)_2PON_3$ and triethylamine to form the acyl azide followed by heating in an inert solvent, such as in warm toluene, preferably at about 80 °C for about three hours, to afford the isocyanate derivative.

The 2(R)-mono-substituted succinates can be prepared by the procedure described in U. S. Serial No. 07/299,696, filed January 23, 1989, which is incorporated herein by reference.

The present invention resides in the discovery that the isocyanate thus produced can be treated directly with a primary or secondary alcohol (R″OH) in the presence of a catalytic amount of a Lewis base such as, for example, dimethylaminopyridine or its equivalent, to afford unexpected high yield of the corresponding carbamate-protected β-amino acid ester represented by the formula:

$$\begin{array}{l} \qquad\quad O \qquad\qquad R' \\ \qquad\quad \| \qquad\qquad \nabla \\ R''O-C-HN-CH_2-CH-CO_2R \end{array}$$

wherein R, R′ and R″ are the same as defined above.

Suitable primary and secondary alcohols include those represented by the formula R″OH wherein R″ represents substituted and unsubstituted alkyl, cycloalkyl and aryl radicals, as well as suitable equivalents such as, for example, silyl radicals. Preferably, the primary and secondary alcohols are those wherein R″ represents substituted and unsubstituted, straight chain as well as branched chain, alkyl radicals having from 1 to about 12 carbon atoms, substituted and unsubstituted cycloalkyl radicals having from 4 to about 7 carbon atoms, and substituted and unsubstituted aryl, alkaryl and aralkyl radicals. Examples of such suitable alcohols include benzyl alcohol, isopropyl alcohol, 4-methoxybenzyl alcohol, 2-trimethylsilylethanol, fluorenyl methanol and benzhydrol. Preferred alcohols are benzyl alcohol and 4-methoxybenzyl alcohol. Other primary and secondary alcohols suitable for use in the practice of the present invention will be readily apparent to those skilled in the art.

The ester derivative is then saponified by any one of numerous well-known procedures, such as by treatment with aqueous lithium hydroxide/THF (tetrahydrofuran), preferably for three hours at 0° C. The resultant product is the corresponding carbamate-protected β-amino acid represented by the formula:

$$\begin{array}{l} \qquad\quad O \qquad\qquad R' \\ \qquad\quad \| \qquad\qquad \nabla \\ R''O-C-HN-CH_2CH-CO_2H \end{array}$$

wherein R′ and R″ are the same as defined above. These are subsequently deprotected by any one of several well-known procedures, such as by acid catalyzed hydrolysis or by hydrogenolysis, to produce the corresponding deprotected β-amino acids represented by the formula:

$$\begin{array}{l} \qquad\qquad\quad R' \\ \qquad\qquad\quad \nabla \\ H_2N-CH_2-CH-CO_2H \end{array}$$

wherein R′ is the same as defined above.

Contemplated equivalents of the general formulas set forth above for the β-amino acids as well as the intermediates are compounds otherwise corresponding thereto and having the same general properties wherein one or more of R, R′ and/or R″ are simple variations of the substituents as defined therein, e.g., wherein R′ is a higher alkyl group. In addition, where a substituent is designated as, or can be, a hydrogen, the exact chemical nature of a substituent which is other than hydrogen at that position is not critical so long as it does not adversely affect the overall synthesis procedure in terms of yield.

The chemical reactions described above are generally disclosed in terms of their broadest application to the preparation of the compounds of this invention. Occasionally, the reactions may not be applicable as described to each compound included within the disclosed scope. The compounds for which this occurs will be readily recognized by those skilled in the art. In all such cases, either the reactions can be successfully performed by conventional modifications known to those skilled in the art, e.g., by appropriate protection of interfering groups, by changing to alternative conventional reagents, by routine modification of reaction conditions, and the like, or other reactions disclosed herein or otherwise conventional, will be applicable to the preparation of the corresponding compounds of this invention. In all preparative methods, all starting materials are known or readily preparable from known starting materials.

Without further elaboration, it is believed that one skilled in the art can, using the preceding description, utilize the present invention to its fullest extent. The following preferred specific embodiments are, therefore, to be construed as merely illustrative, and not limitative of the remainder of the disclosure in any way whatsoever.

In the following examples, melting points were determined on a Fisher-Johns melting point apparatus and are uncorrected. Infrared spectra were measured on an IBM IR30 instrument, absorbance positions are reported in cm-1. Proton and carbon magnetic resonance spectra were recorded on a Varian VXR-300 spectrometer using tetramethylsilane as internal standard. Liquid chromatography was performed on a Spectra Physics chromatography system. All instruments were utilized according to the manufacturer's directions.

## EXAMPLE 1

Preparation of Methyl N-para-methoxy benzyloxycarbonyl-L-$\beta$-phenylalanate: A 100 mL 14/20 round bottomed flask equipped with magnetic stir bar, thermometer adapter, serum cap, reflux condenser, and nitrogen inlet was charged with 2(R)-benzyl mono-methyl succinate (4.85g, 21.8 mmol), triethylamine (3.44g, 33.4 mmol) and toluene 27 mL. The solution was warmed to 90° C with an oil bath and then treated with diphenylphosphoryl azide (9.17g, 33.3 mmol) in 5mL of toluene dropwise via syringe at such a rate that the evolution of nitrogen did not become too vigorous. After the addition was complete the solution was stirred at 90° C until the evolution of nitrogen had completely ceased, ca. 1.5-2h. The solution was then treated with para-methoxybenzyl alcohol (3.05g, 22 mmol) in 5mL of toluene via syringe and the solution stirred at 90° C for an additional 1.5h. The solution was cooled to room temperature, diluted with ethyl acetate and poured into a separatory funnel then washed with 0.2N HCl, saturated aqueous $NaHCO_3$, and brine. The organic solution was dried over anhydrous $MgSO_4$, filtered and concentrated on a rotary evaporator to give an oil, 8.3g. The crude material was purified by preparative HPLC using a Waters Prep 500 utilizing a single silica gel pack and 10% ethyl acetate in hexanes (flow rate 500mL $min^{-1}$). The appropriate fractions were combined and concentrated to give the pure $\beta$-amino acid methyl ester as an oil (4.11g, 53%). $^1H$ NMR ($CDCl_3$) 2.83(1H, m), 2.97(2H, m), 3.34(1H, m), 3.66(3H, s), 3.82(3H, s), 5.05(2H, s), 5.10(1H, m), 6.90(2H, d, $J_{AB}$ = 8.7Hz), 7.24(7H, m), TLC 30% ethyl acetate/hexane $R_f$ = 0.57.

Hydrolysis of N-Moz-$\beta$-Phe-OMe with Lithium Hydroxide; Preparation of N-Moz-$\beta$-Phe-OH: The purified product from the above preparation (4.11g, 11.5 mmol) was dissolved in tetrahydrofuran (70mL) and placed in a one-necked round bottomed flask equipped with a magnetic stir bar and nitrogen inlet. The stirring solution was then treated with 23mL of 0.5 M lithium hydroxide solution all at once. The mixture was stirred until all of the starting material was gone as judged by TLC, ca. 2h. The solution was then concentrated on rotary evaporator, acidified to pH = 1 with 0.2N HCl, and extracted with three portions of ethyl acetate, the combined ethyl acetate solution was washed with brine and dried over anhydrous $MgSO_4$. The drying agent was removed by filtration and the solution concentrated to give a white solid, 3.83g, that was recrystallized from ethyl acetate/hexane to give the pure amino acid, mp 106-107° C, (3.32g 87%).[$\alpha$]D = -1.7 (c = 2.235, EtOH). $^1H$ NMR (acetone-$d_6$) 2.92(3H, m), 3.39 (2H, m), 3.80(3H, s), 5.02(2H, s), 6.40(1H, m), 6.92 (2H, d, $J_{AB}$ = 8.7 Hz), 7.27(5H, m), 7.32(2H, d, $J_{AB}$ = 8.7 Hz). CI mass spectrum m/e 344(2), 300(9), 188(8), 149(8), 133(9), 123(13), 121(100). Anal. Calcd for $C_{19}H_{21}NO_5$ : C,66.46; H,6.16; N,4.07. Found: C,66.67; H,644; N,3.93.

## EXAMPLE 2

The carbamate-protected chiral $\beta$-amino acids set forth in Table 1 were synthesized according to the procedure set forth in Example 1.

TABLE 1

```
    O      R'
    ||     ∇
R"OCH2C-HNCH2CHCO2H
```

| | R' | R" | mp (°C) | $[\alpha]_D$(°C)[1] | Yield % |
|---|---|---|---|---|---|
| a. | $C_6H_5CH_2$ | $CH_3OC_6H_4CH_2$ | 106-107 | -1.7(20) | 70 |
| b[2]. | H | $(CH_3)_3C$ | 58-59.5 | | 23 |
| c[3]. | $(CH_3)_2CHCH_2$ | $CH_3OC_6H_4CH_2$ | 59-61 | -7.6(20) | 92 |
| d. | $CH_3$ | $C_6H_5CH_2$ | 100-102 | -24.5(20) | 84 |
| e[3]. | $(CH_3)_2CH$ | $CH_3OC_6H_4CH_2$ | 97-98 | racemic | 57 |
| f[3] | H | $C_6H_5CH_2$ | 105-107 | | 78 |
| g[3]. | $CH_3$ | $CH_3OC_6H_4CH_2$ | 97-98 | -9.8(23) (Methanol) | 87-92 |
| h[3]. | $(CH_3)_2CHCH_2$ | $C_6H_5CH_2$ | 58-59 | | |
| i[3]. | $C_6H_{11}CH_2$ | $CH_3OC_6H_4CH_2$ | 114-115 | -0.9(24) (Methanol) | 80 |

[1]. Measured in ethyl acetate unless otherwise indicated.
[2]. Comparative
[3]. Analytical data for the carbamate-protected β-amino acid appears below.

3(c) N-para-Methoxy benzyloxycarbonyl-β-L-leucine:
$^1$H NMR (acetone-$d_6$) 0.92(6H, d, J=6.6 Hz), 1.32(1H, m), 1.52(1H, m), 1.67(1H, m), 2.71(1H, m), 3.30(2H, m), 3.80(3H, s), 5.00(2H, s), 6.32(1H, m), 6.90(2H, d, J=8.5 Hz), 7.30(2H, d, J=8.5 Hz). EI mass spectrum m/e 309(38), 137(95), 121(100), 110(32), 91(44), 78(49).

3(e) N-para-Methoxy benzyloxycarbonyl-β-D,L-valine:
$^1$H NMR (acetone-$d_6$) 0.98(3H, d, J=6.6 Hz), 1.01(3H, d, J=6.6 Hz), 1.95(1H, sept, J=6.6 Hz), 2.49(1H, m), 3.80(3H, s), 4.99(2H, s), 6.24(1H, m), 6.91(2H, d,

3(f) N-Carbobenzyloxcarbonyl-β-L-alanine:
$^1$H NMR ($CDCl^3$) 1.24(3H, d, J=7.2 Hz). 2.76(1H, m), 3.38(2H, m), 5.11(2H, s), 5.27(1H, m), 7.37(5H, m). EI mass spectrum me 237932), 219(11), 149(27), 146(29), 130(11), 128(13), 108(83), 107(36), 92(14), 91(100),

79(18), 77(12, 65(22).

3(g) N-para-Methoxybenzyloxycarbonyl-β-L-alanine:

$^1$H NMR (acetone-d$^6$) 1.16(3H, d, J=7.2 Hz), 2.70(1H, m), 3.31(2H, m), 3.80(3H, s), 4.99(2H, s), 6.92(2H, d, J=8.7 Hz), 7.13(2H, d, J=8.7 Hz). EI mass spectrum m/e 267(190, 138(31), 137(30), 121(100), 109(10), 91-(10), 77(10). E1 mass spectrum m/e 295(14), 139(42, 137(59), 121(100) 91(20), 78(19), 70(17), 68(23).

3(h) N-Carbobenzyloxycarbonyl-β-L-Leucine

$^1$H NMR (CDCl$_3$)300 MHZ, 6.71 and 5.10 (1H, brm), 3.30(2H,m), 2.70(1H,m), 1.70 (1H,M), 1.55(1H,m), 1.50 and 1.46 (9H,S), 1.30(1H,m), 0.94 (d,J=6.6H2, 6H). mp=58.0-59.5° C; $[\alpha]_D^{23}$=-8.5° (C=1.05, MeOH).

3(i) N-para-Methoxybenzyloxycarbonyl β-L-cyclohexylmethylalanine

$^1$H NMR (acetone D6) 300 MHZ, 7.31(d,J=8.6 Hz, 2H), 6.92(α, J=8.6 Hz, 2H), 6.31 (1H,m), 5.00 (2H,S), 3.80 (3H,S), 3.30 (2H,m), 2.75 (1H,m), 1.87-1.70 (6H,m), 1.54 (1H,m), 1.37 (2H,m), 1.2 (3H,m), 0.92 (2H,m); mp = 114-115° C; $[\alpha]_D^{24}$ = -0.9 (C=2.575, MeOH).

It is contemplated that carbamate-protected β-amino acids containing other R′ and R″ groups as defined above can be synthesized according to this procedure with similar yields.

This Example 2 illustrates the unexpectedly improved yields utilizing primary and secondary alcohols, as opposed to tertiary alcohols. Ninomita et al, Tetrahedron Lett., 30, 2152-57 (1975) teach that utilization of t-butyl alcohol in the Curtius rearrangement of benzoic acid with diphenoxyphosphoryl azide (DPPA) followed by alcohol treatment gives yields which are superior to ethanol, phenol and benzyl alcohol. However, as can be seen from this Example 2, for preparing β-amino esters, utilization of primary alcohols produces yields very much greater than when t-butyl alcohols are utilized. Furthermore, it is contemplated that similar unexpected results will be obtained utilizing secondary alcohols in place of the primary alcohols of this Example 2.

Ninomita et al, Chem. Pharm. Bull., 22, 1398 (1974) also teach that treatment of monoesters of malonic acid with DPPA/triethylamine in the presence of benzyl alcohol or t-butyl alcohol gives only the benzyl or t-butyl esters and no carbamate. Ninomita et al further teach that the carbamate is formed only when the alcohol is added after heating the acid with DPPA/triethylamine.

Deprotection of the carbamate-protected β-amino acids can be accomplished by methods well known in the art such as, for example, by acid catalyzed hydrolysis or by hydrogenation (hydrogenolysis).

## EXAMPLE 3

This example illustrates procedures for deprotecting carbamate-protected β-amino acids.

### a) Preparation of β-L-leucine Hydrochloride From Methyl N-carbobenzyloxy β-L-leucine.

A 100 mL 24/40 round bottomed flask equipped with a magnetic stir bar and reflux condenser was charged with methyl N-carbobenzyloxy β-L-leucine (1.10g, 3.75mmol), glacial acetic acid, 20mL, and 6N hydrochloric acid, 20mL. The solution was heated to 90° C (oil bath temperature) for a period of 3.5 hours. The solution was cooled to room temperature poured into a separatory funnel and extracted three times with ether. The aqueous phase was then concentrated in vacuo to give a white solid 500mg, 73% yield. mp 195-197° C. Hnmr ($D_2O$), 300 MHz, 3.10 (m,2H), 2.75 (m,1H), 1.54 (m,2H), 1.32 (m,1H), 0.83 (d, J=6.6Hz,3H), 0.82 (d,J=6.3 Hz,3H).

### b) Preparation of BOC-β-L-leucine from β-L-leucine Hydrochloride

A 50mL Erlenmeyer flask was charged with the amino acid hydrochloride from the above reaction (400mg, 2.2mmol) and 5mL of deionized water. The flask was then treated with 0.1N sodium hydroxide solution until the pH was 9. Tetrahydrofuran, 5mL was then added followed by di-tert-butyl dicarbonate (580mg, 2.65mm0l). The pH was maintained at 9 by the periodic addition of .1N NaOH, after the pH had stabilized the solution was poured into a separatory funnel and extracted with ether. The aqueous phase was acidified with potassium bisulfate and then extracted with three portions of ethyl acetate. The ethyl acetate solution was washed with saturated aqueous sodium chloride, dried over magnesium sulfate, filtered and concentrated on a rotary evaporator to give an oil that crystallized on standing. The material was recrystallized from hexane to give a white crystalline solid, 315mg, 58% isolated yield, mp 56-58° C, [α]-

$_D^{24}$ = -9.0 (c = 0.144, ethyl acetate).

c) Deprotection of N-Cbz-β-L-leucine by Hydrogenolysis:

A Fisher-Porter bottle was charged with N-Cbz-β-L-leucine (270mg, 1.14mmol), glacial acetic acid, 10mL and 50mg of 10% palladium on carbon catalyst. The bottle was flushed several times with nitrogen to displace the air and then several times with hydrogen and finally pressurized to 40 psig with hydrogen. The solution was stirred at room temperature for 16h and then the bottle was opened and the contents filtered through a pad of cellite. The filtrate was then concentrated in vacuo to give a white solid that was immediately converted to the Boc derivative with the above procedure to give 210mg of product after recrystallization from hexane 75% yield, mp 56-58 °C.

## EXAMPLE 4

Curtius Rearrangement of Methyl 2(R)-methylsuccinate: Preparation of Methyl N-Moz-α-methyl β-alanine

A 500 mL three-necked round bottomed flask equipped with a nitrogen inlet, reflux condenser, magnetic stir bar, constant pressure addition funnel, and thermometer adapter was charged with methyl 2(R)-methylsuccinate (26.0g, 0.178 mol), triethylamine (21.6g, .214 mol), and toluene (150 mL). The solution was warmed to 85 °C and then treated dropwise with a solution of diphenylphosphoryl azide (49.0g, 0.178 mol) in toluene (50 mL) over a period of 0.5h. The solution was maintained at that temperature for an additional 1.5h and then the solution was treated with a solution of 4-methoxybenzyl alcohol (25.6g, 0.185mol) in toluene (50 mL). The solution was stirred at 85 °C for an additional 2h and then cooled to room temperature. Most of the solvent was removed on a rotary evaporator and then the residue was diluted with 200 mL of ethyl acetate and washed twice with sodium bicarbonate solution, twice with citric acid solution and once with saturated sodium chloride solution. The organic phase was dried with anhydrous magnesium sulfate, filtered and concentrated in vacuo to give 45.0g, 87% of the desired product. $^1H$ NMR ($CDCl_3$) 300MHz 7.32(d, J = 8.4Hz, 2H), 6.91(d, J = 8.4Hz, 2H), 5.2(brm, 1H), 5.05(s, 2H), 3.83(s, 3H), 3.70(s, 3H), 3.35(m, 2H), 2.70(m, 2H), 1.20(d, J = 7.2Hz, 3H).

Hydrolysis of Methyl N-Moz-α-methyl β-alanine: Preparation of α-methyl β-alanine Hydrochloride

A 1000 mL three-necked round bottomed flask equipped with a reflux condenser, nitrogen inlet and mechanical stirrer was charged with methyl N-Moz-α-methyl β-alanine (45.0g, 0.16mol), glacial acetic acid (200mL) and 12N hydrochloric acid (400mL). The solution was then heated to reflux for 3h. After the solution had cooled to room temperature the aqueous phase was decanted from organic residue and the aqueous phase concentrated on a rotary evaporator. Upon addition of acetone to the concentrated residue, a slightly yellow solid formed that was slurried with acetone and the white solid was isolated by filtration on a Buchner funnel. The last traces of acetone were removed by evacuation to give 19.1g, 87% of pure product, mp 128.5-130.5 °C $[\alpha]_D$ @ 25 °C = 9.0° (c = 2.535, Methanol). $^1H$ NMR ($D_2O$) 300 MHz 3.29 (dd, J = 8.6, 13.0Hz, 1H), 3.16 (dd, J = 5.0, 13.0 Hz, 1H), 2.94 (ddq, J = 7.2, 5.0, 8.6 Hz, 1H), 1.30 (d, J = 7.2 Hz, 3H); $^{13}C$ NMR ($D_2O$) 180.84, 44.56, 40.27, 17.49.

Preparation of N-Boc α-Methyl β-Alanine

A solution of α-methyl β-alanine hydrochloride (16.7g, 0.12 mol) in water (120 mL) and tetrahydrofuran (240 mL) was treated with 1N sodium hydroxide solution (120 mL) followed by di-tert-butyl pyrocarbonate (28.8g, 0.132 mol). The pH of the solution was maintained between 9.0-9.5 by the periodic addition of 1N NaOH solution. After 2.5h the pH had stabilized and the reaction was judged to be complete. The excess di-tert-butyl pyrocarbonate was removed by extraction with ether and then the aqueous solution was acidified with 1N $KHSO_4$ to pH = 2. The solution was then extracted four times with ethyl acetate. The combined ethyl acetate extract was washed with saturated aqueous sodium chloride, dried over anhydrous magnesium sulfate, filtered and concentrated on a rotary evaporator to give a thick oil that was stirred with n-hexane whereupon crystals of pure product formed, 19.74g, 80%, mp79-80°C, $[\alpha]_D$ @ 25°C=11.6° (c=1.07, EtOH). $^1$H NMR (acetone $D_6$) 300MHz 6.05(brs 1H), 3.35(m, 1H), 3.21(m, 1H), 2.50(m, 1H), 1.45(s, 9H), 1.19(d, J=7.3Hz, 3H); $^{13}$C NMR (acetone $D_6$) 177.01, 79.28, 44.44, 40.92, 29.08, 15.50.

## EXAMPLE 5

### Preparation of N-(4-Methoxybenzyloxycarbonyl)4-Benzyloxy-β-L-Tyrosine

A solution of methyl 2(R)[4-benzyloxy]benzyl succinate (1.72g, 5.24mmol) and triethylamine (0.85g, 8.41mm0l, 1.6 equivalents) in dry toluene (7.5mL) was warmed to 85°C in an oil bath and then treated with a solution of diphenylphosphoryl azide (1.44g, 5.24mm0l) via syringe over ca. 5 min. The solution was stirred at 85°C for an additional 35 min. until the evolution of nitrogen ceased. The solution was then treated with 4-methoxybenzyl alcohol (0.72g, 5.24mmol) and the solution stirred for 1.5h. The solution was diluted with ethyl acetate and transferred to a separatory funnel and the solution washed with sodium bicarbonate solution, 0.2N HCl, saturated aqueous NaCl and dried over anhydrous $MgSO_4$. The drying agent was removed by filtration and the solvent removed on a rotary evaporator to give 2.67g. This material was dissolved in tetrahydrofuran (50mL) and water (10mL) and treated with lithium hydroxide (0.25g, 5.98mm0l). This solution was stirred at room temperature for 16h. The solution was concentrated on a rotary evaporator and the residue was acidified with 1N $KHSO_4$ and extracted with ethyl acetate. The combined ethyl acetate extracts were washed with saturated aqueous NaCl solution, dried over anhydrous $MgSO_4$, filtered and concentrated to give 2.5g of crude product. The crude product was recrystallized from hexane/ethyl acetate to give 1.95g of pure product, 83%, mp109-111°C, $[\alpha]_D$ @ 23°C = +1.9° (c=1.17, MeOH). $^1$H NMR (acetone-$D_6$) 300 MHz 7.55-7.33(m, 7H), 7.23(d, J=8.4Hz, 2H), 6.98(m, 4H), 6.37(brm, 1H), 5.14(s, 2H), 5.04(s, 2H), 3.85(s, 3H), 3.40(m, 2H), 3.04-2.80(m, 3H). Elemental analysis calc'd. for $C_{26}H_{27}NO_6$: C, 69.48; H, 6.05; N, 3.11. Found: C, 69.19; H, 6.05, N, 3.08.

The preceding examples can be repeated with similar success by substituting the generically or specifically described reactants and/or operating conditions of this invention for those used in the preceding examples.

From the foregoing description, one skilled in the art can easily ascertain the essential characteristics of this invention, and without departing from the spirit and scope thereof, can make various changes and modifications of the invention to adapt it to various usages and conditions.

## Claims

1. Method of preparing a β-amino acid comprising reacting an isocyanate derivative of a 2(R)-mono-substituted succinate with an alcohol selected from the group consisting of primary and secondary alcohols to produce a carbamate-protected β-amino ester, saponifying said ester to produce the corresponding carbamate-protected β-amino acid and deprotecting said carbamate-protected β-amino acid.

2. Method of Claim 1 wherein the isocyanate derivative is obtained by effecting Curtius rearrangement of the 2(R)-mono-substituted succinate.

3. Method of Claim 1 wherein the primary and secondary alcohols are represented by the formula R″OH wherein R″ represents substituted and unsubstituted alkyl, cycloalkyl, silyl, aryl, alkaryl and aralkyl radicals.

4. Method of Claim 3 wherein R″ represents substituted and unsubstituted alkyl radicals having from 1 to about 12 carbon atoms, substituted and unsubstituted cycloalkyl radicals having from about 4 to about 12 carbon atoms, and substituted and unsubstituted aryl radicals.

5. Method of Claim 1 wherein said primary and secondary alcohols are selected from the group consisting of benzyl alcohol, isopropyl alcohol, 4-methoxybenzyl alcohol, 2-trimethylsilylethanol, fluorenyl methanol and benzhydrol.

6. Method of Claim 1 wherein said alcohols are selected from the group consisting of benzyl alcohol and 4-methoxybenzyl alcohol.

7. Method of Claim 1 wherein said carbamate-protected β-amino ester is represented by the formula:

$$\begin{array}{ccccc} & O & & R' & \\ & \| & & \nabla & \\ R''O- & C & -NH-CH_2- & CH & -CO_2R \end{array}$$

wherein R and R′ independently represent substituted and unsubstituted alkyl, cycloalkyl, aryl, aralkyl and alkaryl radicals, and R″ represents radicals derived from said alcohol.

8. Method of preparing a carbamate-protected β-amino ester of the formula:

$$\begin{array}{ccccc} & O & & R' & \\ & \| & & \nabla & \\ R''O- & C & -NH-CH_2- & CH & -CO_2R \end{array}$$

wherein R and R′ independently represent substituted and unsubstituted alkyl radicals having from 1 to about 12 carbon atoms, substituted and unsubstituted cycloalkyl radicals having from about 4 to about 7 carbon atoms, and substituted and unsubstituted aryl, aralkyl and alkaryl radicals and R″ represents radicals derived from a primary or a secondary alcohol, said method comprising:

a) effecting Curtius rearrangement of a 2(R)-mono-substituted succinate to produce the corresponding isocyanate derivative thereof; and

b) reacting the isocyanate derivative with a primary or secondary alcohol to produce the corresponding carbamate-protected β-amino acid ester.

9. Method of preparing a carbamate-protected β-amino acid of the formula:

$$\begin{array}{ccccc} & O & & R' & \\ & \| & & \nabla & \\ R''O- & C & -NH-CH_2- & CH & -CO_2H \end{array}$$

wherein R′ represents substituted and unsubstituted alkyl radicals having from 1 to about 12 carbon atoms, substituted and unsubstituted cycloalkyl radicals having from about 4 to about 7 carbon atoms, and substituted and unsubstituted aryl, alkaryl and aralkyl radicals, and R″ represents radicals derived from a primary or a secondary alcohol, said method comprising saponifying the carbamate-protected β-amino ester of Claim 8.

10. Method of preparing a β-amino acid of the formula

$$\begin{array}{ccc} & R' & \\ & \nabla & \\ H_2N-CH_2- & CH & -CO_2H \end{array}$$

wherein R′ represents substituted and unsubstituted alkyl radicals having from about 4 to about 7 carbon atoms, and substituted and unsubstituted aryl, aralkyl and alkaryl radicals, said method comprising deprotecting the carbamate-protected β-amino acid of Claim 9.

11. Carbamate-protected β-amino ester of the formula:

$$\begin{array}{ccccc} & O & & R' & \\ & \| & & \nabla & \\ R''O- & C & -NH-CH_2- & CH & -CO_2R \end{array}$$

wherein R and R′ independently represent substituted and unsubstituted alkyl radicals having from 1 to about 12 carbon atoms, substituted and unsubstituted cycloalkyl radicals having from about 4 to about 7 carbon atoms, and substituted and unsubstituted aryl, alkaryl and aralkyl radicals and R″ represents radicals derived from a primary or a secondary alcohol.

12. β-amino ester of Claim 11 wherein said R″ represents substituted and unsubstituted alkyl radicals

having from 1 to about 12 carbon atoms, substituted and unsubstituted cycloalkyl radicals having from about 4 to about 7 carbon atoms, and substituted and unsubstituted silyl, aryl, alkaryl and aralkyl radicals.

13. β-Amino ester of Claim 11 wherein said $R''$ represents a radical derived from an alcohol selected from the group consisting of benzyl alcohol, isopropyl alcohol, 4-methoxybenzyl alcohol, 2-trimethylsilylethanol, fluorenyl methanol and benzhydrol.

14. β-Amino ester of Claim 11 wherein said $R''$ represents radicals derived from a primary alcohol.

15. β-Amino ester of Claim 14 wherein said primary alcohol is selected from the group consisting of benzyl alcohol and 4-methoxybenzyl alcohol.

16. Carbamate-protected β-amino acid represented by the formula:

$$R''O-\overset{\overset{O}{\|}}{C}-NH-CH_2-\overset{\overset{R'}{\nabla}}{C}H-CO_2H$$

wherein $R'$ represents substituted and unsubstituted alkyl radicals having from 1 to about 12 carbon atoms, substituted and unsubstituted cycloalkyl radicals having from about 4 to about 7 carbon atoms, and substituted and unsubstituted aryl, aralkyl and alkaryl radicals, and $R''$ represents radicals derived from a primary or a secondary alcohol.

17. β-Amino acid of Claim 16 wherein said $R''$ represents a radical derived from an alcohol selected from the group consisting of benzyl alcohol, isopropyl alcohol, 4-methoxybenzyl alcohol, 2-trimethyl silylethanol, fluorenyl methanol and benzhydrol.

18. β-Amino acid of Claim 16 wherein said $R''$ represents radicals derived from a primary alcohol.

19. β-Amino acid of Claim 18 wherein said primary alcohol is selected from the group consisting of benzyl alcohol and 4-methoxybenzyl alcohol.

20. In a method for preparing β-amino acids wherein an isocyanate derivative of an amino acid ester is reacted with a tertiary alcohol to produce a carbamate-protected β-amino ester which is subsequently saponified and then deprotected, the improvement which comprises reacting an isocyanate derivative of a 2-(R)-mono-substituted succinate with an alcohol selected from the group consisting of primary and secondary alcohols represented by the formula $R''OH$ wherein $R''$ represents substituted and unsubstituted alkyl radicals having from 1 to abut 12 carbon atoms, substituted and unsubstituted cycloalkyl radicals having from about 4 to about 7 carbon atoms and substituted and unsubstituted silyl, aryl, alkaryl and aralkyl radicals.